# EUROPEAN PATENT APPLICATION

(11) **EP 1 428 529 A1**
(43) Date of publication of application: **16.06.2004**
(21) Application number: 02765385.6
(22) Date of filing: 30.08.2002
(51) Int. Cl.: A61K 31/343, A61P 3/06, A61P 9/10, A61P 43/00, C07D 307/79

(54) **APOLIPO PROTEIN E SECRETION PROMOTERS**

(30) Priority: 31.08.2001 JP 2001263547
(71) Applicant: CHUGAI SEIYAKU KABUSHIKI KAISHA, Tokyo, 115-8543 (JP)
(72) Inventor: YOKOYAMA, Shinji, Nagoya-shi, Aichi 467-0024 (JP); KIM, Myungjoon c/o Chugai Seiyaku Kabushiki Kaisha, Gotenba-shi, Shizuoka 412-8513 (JP); KAWABE, Yoshiki Chugai Seiyaku Kabushiki Kaisha, Gotenba-shi, Shizuoka 412-8513 (JP); CYNSHI, Osamu c/o Chugai Seiyaku Kabushiki Kaisha, Gotenba-shi, Shizuoka 412-8513 (JP)
(74) Representative: VOSSIUS & PARTNER
(86) International application number: PCT/JP2002/008807
(87) International publication number: WO 2003/018001

(57) **Abstract**

A pharmaceutical composition comprising a compound represented by the following formula: 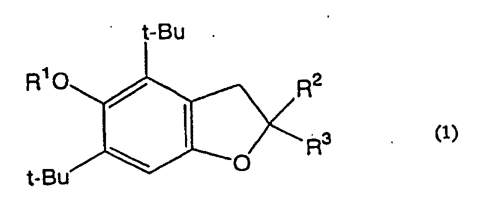 which promote the secretion of ApoE, lower the onset frequency of acute coronary syndrome or relieve the symptom thereof, and lower intracellular lipid conten; and a method therefor are provided. Moreover, a use of a compound of formula (1) in manufacturing the above pharmaceutical composition is also provided.

## Description

### FIELD OF THE INVENTION

The present invention relates to a pharmaceutical composition for promoting the secretion of apolipoprotein E (hereinafter referred to as ApoE), to a method for promoting the same, and to a use of a compound in manufacturing such a pharmaceutical composition. More specifically, this invention relates to a pharmaceutical composition for promoting the secretion of ApoE comprising a certain 4,6-di-t-butyl-dihydrobenzofuran derivative, to a method for promoting the same, and to a use of certain 4,6-di-t-butyl-dihydrobenzofuran derivatives in manufacturing such a pharmaceutical composition.

### BACKGROUND OF THE INVENTION

ApoE is one of the major constituents of apolipoproteins contained in lipoproteins such as chylomicron, very low density lipoprotein (VLDL), and high density lipoprotein (HDL). It is a glycoprotein composed of 299 amino acid residues and some sugar moieties containing sialic acids and has a molecular weight of approximately 34,000. ApoE is produced in kidney, brain, macrophages and the like, and mainly in liver.

Apolipoproteins contained in lipoproteins are believed to be important to the binding of the lipoproteins to their respective receptors. Therefore, ApoEs can bind to a variety of receptors, such as low density lipoprotein (LDL) receptor, VLDL receptor, LDL receptor-related protein, and ApoE receptor 2, which receptors are present on cell surfaces throughout a living body. Hence, as a result of the interaction of ApoEs contained in chylomicrons, VLDLs and HDLs with the corresponding receptors, the lipoproteins are delivered to all the organs of a living body where the lipoproteins are decomposed into triglycerides and cholesterol (hereinafter the term "cholesterol" includes both the free and ester forms of cholesterol). See, Davignon J, Cohn JS, Mabile L, Bernier L, "Apolipoprotein E and atherosclerosis: insight from animal and human studies", Clinica Chimica Acta. 286 (1-2):115-43 (1999); Rubin. E.M. and Plump. A.S., p.199 in Lipoproteins in health and disease, Ed. Betteridge, D.J., Illingworth. and Shepherd. J. (1999) Arnold.

ApoEs are also known to be essential for a normal lipid metabolism of macrophages. Further, ApoEs are believed to play an important role in the reverse cholesterol transport from peripheral tissues to liver. Furthermore, it is known that ApoEs produced in macrophages or hepatic cells undergo a saccharification and, consequently, most of them remain bound to the cells and are not secreted. See, Schmitt M, Grand-Perret T, "Regulated turnover of a cell surface-associated pool of newly synthesized apolipoprotein E in HepG2 cells", Journal of Lipid Research. 40(1):39-49 (1999); Zhao Y, Mazzone T, "Transport and processing of endogenously synthesized ApoE on the macrophage cell surface", Journal of Biological Chemistry, 275(7):4759-65 (2000).

Advances have been made in recent years in embryological engineering and various types of transgenic mice have been developed, such as ApoE deficient mouse. A normal mouse is inherently strongly resistant to arteriosclerosis. However, an ApoE deficient mouse is known to be susceptible to type III hyperlipidemia due to the deletion of a single gene coding for ApoE, which disease then spontaneously progresses to atherosclerosis characterized by the lipid accumulation in arterial wall. Atherosclerosis is characterized in that lipids are deposited on the inner walls of arteries.

It has been observed that introduction of ApoE-producing macrophages obtained from a wild type mouse into an ApoE deficient mouse via a bone marrow transplantation inhibits lipid deposition on inner walls of arteries of the ApoE deficient mouse. See, Linton MF, Atkinson JB, Fazio S. "Prevention of atherosclerosis in apolipoprotein E-deficient mice by bone marrow transplantation." Science 267:1034-1037 (1995); Boisvert W.A., Spangenberg J. & Curtiss L.K., "Treatment of severe hypercholesterolemia in apolipoprotein E-deficient mice by bone marrow transplantation." J. Clin. Invest. 96, 1118-1124 (1995); Bellosta S, Mahley RW, Sanan DA, Murata J, Newland DL, Taylor JM, Pitas RE., "Macrophage-specific expression of human apolipoprotein E reduces atherosclerosis in hypercholesterolemic apolipoprotein E-null mice.", J Clin Invest 96:2170-2179 (1995).

It has also been observed that transplantation of macrophages obtained from an ApoE deficient mouse to a wild type mouse promotes a lipid deposition on inner walls of arteries of the wild type mouse. See, Fazio S, Babaev V R, Murray, A B, Hasty A H, Carter K J, Gleaves L A, Atkinson J B & Linton M F, "Increased atherosclerosis in mice reconstituted with apolipoprotein E null macrophages", Proc. Natl. Acad. Sci. USA, 94, 4647-4652 (1997).

These results were obtained even in the cases where no influence of the transplantation on the hyperlipidemic condition was observed, suggesting that the secretion of ApoE from macrophages is a more crucial factor than the distribution of serum lipoprotein in inhibiting lipid deposition on inner walls of arteries.

Cholesterol efflux capacity of serum which has been lost due to ApoE-deficiency may be restored by a low-dose expression of ApoE in macrophages. See, Yenhong Zhu, Stefano Bellosta, Claus Langer, Franco Bernini, Robert E. Pitas, Robert W. Mahley, Gerd Assmann, and Arnold von Eckardstein, "Low-dose expression of a human apolipoprotein E transgene in macrophages restores cholesterol efflux capacity of apolipoprotein E-deficient mouse plasma", PNAS 95: 7585-7590 (1998). ApoE-secretion from macrophages is essential for the promotion of cholesterol efflux. See, Lin CY, Duan H, Mazzone T, "Apolipoprotein E-dependent cholesterol efflux from macrophages: kinetic study and divergent mechanisms for endogenous versus exogenous apolipoprotein E", Journal of Lipid Research. 40(9):1618-27 (1999). Hence, it is believed that ApoE secreted from macrophages plays an important role in the aforementioned inhibitory effect on lipid deposition.

Cholesterol, especially free cholesterol, not only is an important constituent of plasma membrane but also plays an important role as a constituent of organelle membrane in cells. However, due to the interference with cell functions made by the presence of excess free cholesterol, the excess free cholesterol is esterified by acyl CoA-cholesterol acyltransferase and then stored as cholesteryl ester in the cells. In the case where cells are macrophages, they accumulate large excesses of cholesteryl ester therein due to the expression of scavenger receptors by the macrophages which is not regulated by sterol regulatory element binding proteins, and such macrophages then become foam cells. The accumulated cholesteryl is hydrolyzed into free cholesterol by neutral cholesterol esterase, as necessary.

It is believed that cholesterol excreted from cells by ApoE is mainly free cholesterol present on the cell membrane. Cellular cholesteryl ester is hydrolyzed into free cholesterol to keep the level of free cholesterol on the cell membrane constant. As a result, the cholesterol content of the cells decreases.

It was believed that acute myocardial infarction is caused by a stenosis of responsible coronary artery. However, recent studies revealed that the proportion of the cases where significant organic stenosis is observed in infract-related coronary artery before the onset of acute myocardial infarction is less than 15 percent, and, in many cases, the cause of acute myocardial infarction is an obstruction in coronary artery resulting from thrombi which have been formed via a disruption of atherosclerotic plaques. It was also revealed that an unstable angina is the case where thrombi formed by the disruption of plaques are transient and do not bring about a myocardial infarction.

A series of diseases caused by the thrombotic obstruction of coronary artery resulting from the disruption of atherosclerotic plaques is collectively called acute coronary syndrome (ACS). See, Davies M J., "Stability and instability: two faces of coronary atherosclerosis", Circulation., 94:2013-2020 (1996); Libby P, "The molecular bases of the acute coronary syndromes", Circulation., 91:2844-2850 (1995); Falk E, Shah P, Fuster V, "Coronary plaque disruption", Circulation., 92:657671 (1995). In view of the cause of ACS, ACS can undoubtedly be inhibited by making it hard to disrupt the atherosclerotic plaques.

It has become evident that the liability of atherosclerotic plaques to be disrupted depends on their composition rather than their size or degree of clustering in coronary artery. One of the most significant features of atherosclerotic plaques which make them liable to be disrupted is that the plaques have lipid-rich cores which are composed of macrophage-derived foam cells and therefore contain a large amount of cholesteryl ester. See, Libby P, Clinton SK., "The role of macrophages in atherogenesis", Curr Opin Lipidol., 4:355-363 (1993). Thus, the removal of cholesteryl ester from the lipid-rich cores or the foam cells will make it hard to disrupt atherosclerotic plaques. In the lipid-rich cores, it is also observed that lipids, especially cholesterol, are accumulated in intercellular space of the foam cells. The lipids thus accumulated are normally taken up by macrophages. Accordingly, the removal of such cholesterol taken up by macrophages will promote the removal of cholesterol deposited in intercellular space, thereby allowing an efficient removal of lipids from lipid-rich cores.

Recently, it has been shown that a reduction in the lipid content of lipid-rich cores results in stabilized atherosclerotic plaques which are hard to disrupt. Aikawa M et al showed that atherosclerotic plaques in an animal were stabilized and made hard to disrupt by giving to the animal a diet containing a lowered content of lipids. See, Aikawa M, Rabkin E, Okada Y, Voglic SJ, Clinton SK, Brinckerhoff CE, Sukhova GK, Libby P, "Lipid lowering by diet reduces matrix metalloproteinase activity and increases collagen content of rabbit atheroma: a potential mechanism of lesion stabilization. Circulation", 97(24):2433-44 (1998). Further, Crisby et al showed that a significant decrease in serum cholesterol was achieved by administering antihyperlipidemic agent, HMG-CoA reductase inhibitor, thereby atherosclerotaic plaques being stabilized and made hard to disrupt. See, Crisby M, Nordin-Fredriksson G, Shah P. K., Yano J., Zhu J., Nilsson J.(2001), "Pravastatin Treatment Increases Collagen Content and Decreases Lipid Content, Inflammation, Metalloproteinases, and Cell Death in Human Carotid Plaques": "Implications for Plaque Stabilization", Circulation 103: 926-933; Fukumoto Y., Libby P., Rabkin E., Hill C. C., Enomoto M., Hirouchi Y., Shiomi M., Aikawa M, "Statins Alter Smooth Muscle Cell Accumulation and Collagen Content in Established Atheroma of Watanabe Heritable Hyperlipidemic Rabbits". Circulation 103: 993-999 (2001).

Accordingly, if the secretion of ApoE is promoted, whereby the cholesterol efflux capacity of serum is promoted, and if the efflux capacity thus promoted contributes to the removal of cholesterol from atherosclerotic plaques, i.e., to the stabilization of the plaques, it will become possible to prevent and treat ACS such as acute myocardial infarction and unstable angina.

### PRIOR ART

Liver X receptor (LXR) has been reported to have a capability of promoting the secretion of ApoE. However, it has not yet been clinically used. Therefore, there exists a need in the art for a further drug which has an activity increasing a topical or blood ApoE level.

The compounds represented by the formula: 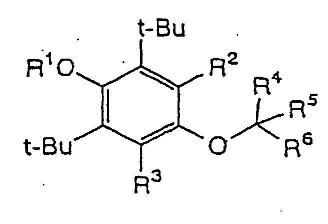 are disclosed in JP 6-206842A, the corresponding US Patent No.5,574,178, and the corresponding European Patent No.0665208B, as having an antioxidant activity.

The same compounds are also disclosed in JP 11-21238A, the corresponding US Patent No.6,156,793, and the corresponding European Patent Appln. No.98917638.3, as active ingredients of a pharmaceutical composition for preventing and treating arteriosclerosis.

However, these publications and patents do not disclose that the compounds have an activity promoting secretion of ApoE or decreasing intracellular lipid content, especially intracellular cholesterol content, via the promoted secretion of ApoE.

### DISCLOSURE OF THE INVENTION

The present invention provides a pharmaceutical composition which can remove intracellular lipids via the promoted secretion of ApoE.

We found that certain 4,6-di-t-butyl-dihydrobenzofuran derivatives promote the secretion of ApoE in cells so that a blood ApoE level or topical ApoE level in an organ is increased, whereby an intracellular lipid content, especially intracellular cholesterol content, is decreased.

Therefore, the present invention provides a pharmaceutical composition for promoting the secretion of ApoE, comprising a compound represented by formula (1): 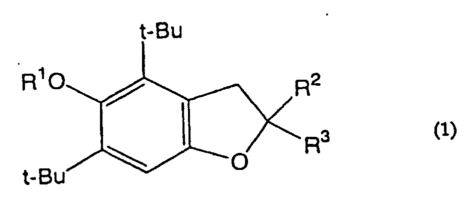 wherein
R¹ represents hydrogen atom, an acyl group or an arylalkoxycarbonyl group, and
R² and R³ are each independently a substituted or unsubstituted alkyl group, a substituted or unsubstituted alkenyl group, or a substituted or unsubstituted alkynyl group, or R² and R³ may combine to form a cycloalkyl group.

In one embodiment, the present invention provides a pharmaceutical composition for increasing a blood ApoE level or topical ApoE level in an organ, whereby an intracellular lipid content is decreased.

In a preferred embodiment, the intracellular lipid is present in atherosclerotic plaques, and more preferably the intracellular lipid is cholesterol present in atherosclerotic plaques.

In a more preferred embodiment, the present invention provides a pharmaceutical composition for removing intracellular cholesterol from atherosclerotic plaques to stabilize atherosclerotic plaques, i.e., to prevent the disruption of atherosclerotic plaques, whereby the risk of acute coronary syndrome such as acute myocardial infarction or unstable angina is reduced or the severity of the disease is alleviated.

The present invention also provides a pharmaceutical composition for reducing the risk of acute coronary syndrome or alleviating the severity of the disease, comprising a compound represented by formula (1).

Further, the present invention provides a pharmaceutical composition for lowering intracellular lipid content, comprising a compound represented by formula (1).

Furthermore, the present invention provides a method for promoting the secretion of ApoE, comprising administering an promoting-effective amount of a compound represented by formula (1) to a patient in need of such promotion.

The present invention also provides a method for reducing the risk of acute coronary syndrome such as acute myocardial infarction or unstable angina or alleviating the severity of the disease, comprising administering a therapeutically effective amount of a compound represented by formula (1) to a patient in need of such treatment.

Further, the present invention provides a method for lowering intracellular lipid content, comprising administering a therapeutically effective amount of a compound represented by formula (1) to a patient in need of such treatment.

Furthermore, the present invention provides a use of a compound represented by formula (1) in manufacturing the foregoing pharmaceutical composition.

### BRIEF DESCRIPTION OF the DRAWING

Fig. 1 shows a Western Blotting of the supernatants of foamed C57BL/6J mouse-derived macrophages untreated or treated with BO-653 and ApoE deficient mouse-derived macrophages untreated with BO-653, obtained by using anti-mouse ApoE antibody.
Fig. 2 shows a SDS-PAGE of lipoprotein-containing fractions prepared from serums taken from individual mice which have been fed a high-fat diet with or without 0.65% BO-653. The lipoprotein-containing fractions were loaded on the gel in such a manner that each lane has lipoprotein whose amount corresponds to that contained in 12.5 µL of serum.

### MOST PREFERRED EMBODIMENTS OF THE INVENTION

R¹ in the compounds of the present invention of formula (1) is hydrogen atom, an acyl group or an arylalkoxycarbonyl group. Preferred acyl groups are those having 1 to 10 carbon atoms, and examples include formyl, acetyl, propionyl and benzoyl groups. Preferred arylalkoxycarbonyl groups are those having 7 to 11 carbon atoms, and examples include benzyloxycarbonyl and naphthylmethoxycarbonyl groups.

R¹ is preferably hydrogen atom or an acyl group, more preferably hydrogen atom or acetyl group, especially hydrogen atom.

R² and R³ in the compounds of formula (1) are each independently a substituted or unsubstituted alkyl group, a substituted or unsubstituted alkenyl group, or a substituted or unsubstituted alkynyl group.

Preferred alkyl groups are straight or branched alkyl groups having 1 to 20 carbon atoms, and examples include methyl, ethyl, n-propyl, isopropyl, n-butyl, isobutyl, s-butyl, t-butyl, n-pentyl, isopentyl, sec-pentyl, t-pentyl, neopentyl, n-hexyl, isohexyl, ethylbutyl, n-heptyl, isoheptyl, ethylpentyl, n-octyl, ethylhexyl, propylpentyl, nonyl, decyl, pentadecyl and stearyl groups. More preferred alkyl groups are straight or branched alkyl groups having 1 to 10 carbon atoms, especially straight alkyl groups having 3 to 8 carbon atoms.

Preferred alkenyl groups are straight or branched alkyl groups having 2 to 20 carbon atoms, and examples include ethenyl, propenyl, isopropenyl, butenyl, isobutenyl, pentenyl, isopentenyl, hexenyl, isohexenyl, ethylbutenyl, heptenyl, isoheptenyl, ethylpentenyl, octenyl, nonenyl, decenyl and pentadecenyl groups. More preferred alkenyl groups are straight or branched alkenyl groups having 2 to 10 carbon atoms, especially straight alkenyl groups having 3 to 8 carbon atoms.

Preferred alkynyl groups are straight or branched alkynyl groups having 2 to 20 carbon atoms, preferably 2 to 10 carbon atoms, especially straight alkynyl groups having 3 to 8 carbon atoms. The examples of alkynyl groups include those corresponding to the examples of alkenyl groups.

R² and R³ may combine to form a cycloalkyl group having 5 to 10 carbon atoms. Preferred examples of cycloalkyl group are cyclopentyl, cyclohexyl, cycloheptyl, cyclooctyl, cyclononyl and cyclodecyl groups.

When R² and R³ are each alkyl, alkenyl or alkynyl group, they may have one or more substituents, and examples include a halogen, a lower alkoxy, hydroxy, amino, nitro and trifluoromethyl groups.

Preferred examples of R² and R³ are straight and unsubstituted alkyl groups having 3 to 8 carbon atoms, and most preferably both R² and R³ are n-pentyl group.

Preferred compounds of formula (1) are as follows:
4,6-di-t-butyl-5-hydroxy-2,2-dimethyl-2,3-dihydrobenzofuran;
4,6-di-t-butyl-5-hydroxy-2,2-diethyl-2,3-dihydrobenzofuran;
4,6-di-t-butyl-5-hydroxy-2,2-di-n-propyl-2,3-dihydrobenzofuran;
4,6-di-t-butyl-5-hydroxy-2,2-di-isopropyl-2,3-dihydrobenzofuran;
4,6-di-t-butyl-5-hydroxy-2,2-di-n-butyl-2,3-dihydrobenzofuran;
4,6-di-t-butyl-5-hydroxy-2,2-di-s-butyl-2,3-dihydrobenzofuran;
4,6-di-t-butyl-5-hydroxy-2,2-di-t-butyl-2,3-dihydrobenzofuran;
4,6-di-t-butyl-5-hydroxy-2,2-di-n-pentyl-2,3-dihydrobenzofuran;
4,6-di-t-butyl-5-hydroxy-2,2-di-t-pentyl-2,3-dihydrobenzofuran;
4,6-di-t-butyl-5-hydroxy-2,2-di-isopentyl-2,3-dihydrobenzofuran;
4,6-di-t-butyl-5-hydroxy-2,2-di-neopentyl-2,3-dihydrobenzofuran;
4,6-di-t-butyl-5-hydroxy-2,2-di-n-hexyl-2,3-dihydrobenzofuran;
4,6-di-t-butyl-5-hydroxy-2,2-di-n-heptyl-2,3-dihydrobenzofuran;
4,6-di-t-butyl-5-hydroxy-2,2-di-n-octyl-2,3-dihydrobenzofuran;
4,6-di-t-butyl-5-hydroxy-2,2-di-n-nonyl-2,3-dihydrobenzofuran; and
4,6-di-t-butyl-5-hydroxy-2,2-di-n-decyl-2,3-dihydrobenzofuran.

Especially preferable compound of formula (1) is 4,6-di-t-butyl-5-hydroxy-2,2-di-n-pentyl-2,3-dihydrobenzofuran.

The compounds of formula (1) used in the present invention can be synthesized according to the procedures described in JP 6-206842A, the corresponding US Patent No.5,574,178, or the corresponding European Patent No.0665208B, for example.

In the present invention, the examples of organs where an ApoE level is topically increased include intima and, in particular, those in which numerous macrophages are located. A blood ApoE level can be determined according to an immunological method in which an anti-ApoE antibody is used. On the other hand, a topical ApoE level in an organ can be determined according to such an immunological method or a molecular biological method such as FISH method in which an ApoE-gene or a fragment thereof is used.

The term "intracellular lipids" as used herein refers to phospholipids, triglycerides, cholesteryl esters, and free cholesterol. In particular, cholesteryl esters and free cholesterol represent main intracellular lipids. The examples of cells in which these lipids are accumulated typically include vascular smooth muscle cells and, in particular, macrophages. The term "removal of intracellular lipids" refers to the event in which intracellular lipids or cell membrane lipids are removed from the cells via the formation of a nascent lipoprotein by the action of ApoE on the lipids.

The term "atherosclerotic plaques" as used herein refers to a vascular lesion formed by a fibrous capsule composed of macrophage-derived foam cells and smooth muscle cells which covers lipid-rich cores.

The term "disruption of atherosclerotic plaques" means that the fibrous capsule composed of smooth muscle cells which covers the lipid-rich cores is disrupted. Due to the disruption, tissue factors expressed in the foam cells and located on their surface are exposed to flowing-blood and then formed into thrombi. When the formation of thrombi occurs in coronary artery, they may cause the onset of acute myocardial infarction or unstable angina.

The term "stabilization of atherosclerotic plaques" as used herein means that the atherosclerotic plaques become hard to disrupt by reducing the lipid content of lipid-rich cores.

The pharmaceutical composition of the present invention may be formulated in various dosage forms depending on the route of administration, by combining a compound represented by formula (1) which is an active ingredient with physiologically acceptable solid or liquid pharmaceutical carriers. The examples of the route of administration include oral route and parenteral routes such as intravenous injection. Further, the composition may be administered as a sustained-release formulation, or topically by means of a catheter. The examples of the pharmaceutical carriers include those commonly used, such as excipients, binders, disintegrants, lubricants, coating agents, dissolution-aids, emulsifiers, suspending agents, stabilizers, fats and oils, and solvents. The examples of the dosage forms include tablets, granules, pills, capsules, solutions, syrups, suspensions, emulsions and injections.

The actual dosage of the compound represented by formula (1) of the present invention will be determined depending on the age of the patient, the severity of the condition to be treated, the route of administration, and the like. However, the dosage will normally fall within 1 - 1000 mg, preferably 50 - 400 mg per day in the treatment of adult human, and can be taken all at once or divided up several times.

The following examples are provided herein for purposes of illustration only, and are not intended to limit the scope of the present invention.

### EXAMPLES

### Test example 1: ApoE secretion-promoting and intracellular cholesterol-removing effects

4.05 w/v% thioglycollate-elicited intraperitoneally exuded cells were prepared from C57BL/6J and ApoE-deficient mice. The cells were inoculated on 12-well plate at 1.0 x 10⁵ cells/well. The cells were cultured at 37°C under 5 vol% CO₂. After 3 hours of culture, the culture medium was changed to a fresh one to remove inadhesive cells, leaving mouse-peritoneal macrophages in each of the wells. Next day, the mouse-peritoneal macrophages were cultured in a medium containing 50 µg of protein per mL of acetylated LDL for 24 hours to form foam cells.

To the wells containing the foamed C57BL/6J mouse-derived macrophages, 4,6-di-t-butyl-2,2-dipentyl-5-hydroxy-2,3-dihydrobenzofuran (BO-653) was added to final concentrations of 0, 3 and 10 µmol/L. After 24 hours of culture, the supernatants were collected to prepare samples for SDS-PAGE.

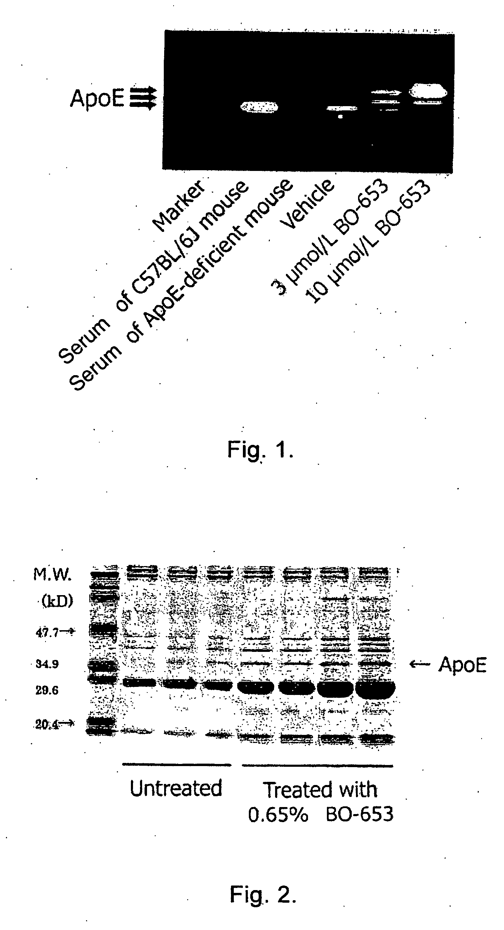

The samples were subjected to 10 w/v% SDS-PAGE. Following the SDS-PAGE, the bands were transferred onto nitrocellulose papers using semi-drying method and blocked with TBS containing 10 w/v% skim milk and 0.1 vol% Tween-20. Rabbit anti-mouse ApoE-antibody (#K23100R, BIODESIGN) and peroxidase-labeled anti-rabbit IgG antibody (#7071-1, Cell Signaling TECHNOLOGY) were used as the first and secondary antibodies, respectively. ApoE was detected using chemoluminescence method (PIERCE). The results are shown in Fig. 1.

As can be seen from Fig. 1, the increased saccharified-ApoE expression was observed in the cultures to which BO-653 was added. In contrast, no saccharified-ApoE was observed in the cultures of macrophages derived from ApoE-deficient mouse.

On the other hand, the foamed macrophages which were cultured in the presence of BO-653 and harvested at the same time as the collection of the supernatants were extracted with a mixed solvent of hexane/2-propanol (3:2 v/v) to obtain lipids. Then, the total amount of cholesterol in the lipids was measured using the enzymatic method described by Yoshiki Kawabe et al., "Oxidation-Induced Aggregation of Rabbit Low-Density Lipoprotein by Azo Initiator", Achieves of Biochemistry and Biophysics 310, 489-496 (1994). We regarded the total amount of cholesterol as intracellular cholesterol content of the macrophages. Table 1 shows percent reduction in intracellular cholesterol content of BO-653-treated macrophages relative to the vehicle used for culturing the macrophages. The data demonstrate a decrease in intracellular cholesterol content due to the treatment with BO-653.

**Table 1:**

| Reduction in Intracellular Cholesterol Content | | |
|---|---|---|
| BO-653 (µmol/L) | Cholesterol Reduction (%) | |
| | Means | S.D. |
| 0 | 100.0 | 6.9 |
| 3 | 132.6 | 23.1 |
| 10 | 145.9 | 28.1 |

### Test example 2: ApoE increasing effect in mouse serum

Seven-week-old C57BL/6J mice were divided into first group of three and second group of four. The animals of the first and second groups were fed a high fat diet containing no BO-653 and 0.65% BO-653, respectively. After 5 weeks of diet initiation, the animals were anesthetized with ether and their blood was individually drawn from vena cava descendens. Serum samples were prepared from the drawn blood and 100 µL of each of the samples was placed into a tube. To the tube, 900 µL of sodium bromide solution having 1.256 density was added, and the tube was centrifuged in TL-100 desk type ultracentrifuge at 100,000 rpm for 20 hours using TLA-100.2 rotor. Lipoprotein-containing fraction thus obtained was collected and treated with 10 w/v% trichloroacetic acid solution. The precipitate was collected and dissolved into 80 µL of solution for SDS-PAGE. 10 µL of the solution was subjected to 15 w/v% SDS-PAGE and the gels were stained with Coomassie brilliant blue. By comparing the bands with molecular weight markers, ApoE was identified. The results are shown in Fig. 2.

As can be seen from Fig. 2, an increased ApoE production was observed in the animals of the second group relative to the first group.

As described previously, the compounds of formula (1) are disclosed in JP 11-21238A and in the corresponding patents issued in other countries as an active ingredient of a pharmaceutical composition for preventing and treating arteriosclerosis. However, in those patents, the compounds of formula (1) are described as inhibitors of the formation of arteriosclerotic lesions which inhibits the formation in a direct manner, as can be seen from the descriptions of their Test Examples. In contrast, according to the present invention, the compounds of formula (1) act via the following mechanism: promotion of the secretion of ApoE, removal of free cholesterol, and then amelioration of peripheral diseases. As a result, it can be expected that non-arteriosclerotic diseases such as acute myocardial infarction and unstable angina will be suppressed with the compounds. Therefore, the pharmaceutical activity of the compounds of formula (1) found by the present inventors is remarkably different from that disclosed in the above patents.

## Claims

1. A pharmaceutical composition for promoting the secretion of apolipoprotein E, comprising a compound represented by formula (1): 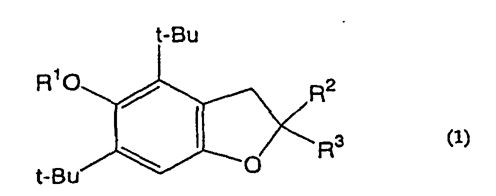 wherein
R¹ represents hydrogen atom, an acyl group or an arylalkoxycarbonyl group, and
R² and R³ are each independently a substituted or unsubstituted alkyl group, a substituted or unsubstituted alkenyl group, or a substituted or unsubstituted alkynyl group, or R² and R³ may combine to form a cycloalkyl group.

2. The composition of Claim 1, wherein said secretion of apolipoprotein E increases a blood apolipoprotein E level or a topical apolipoprotein E level in an organ.

3. The composition of Claim 1, wherein intracellular lipids are removed via the increase in an apolipoprotein E level.

4. The composition of Claim 3, wherein said intracellular lipids are present in atherosclerotic plaques.

5. The composition of Claim 4, wherein said removal of intracellular lipids results in stabilization of atherosclerotic plaques for the prevention of the disruption of atherosclerotic plaques.

6. The composition of Claim 3, wherein said intracellular lipids are free cholesterol or cholesteryl esters.

7. The composition of Claim 5, wherein said prevention of the disruption of atherosclerotic plaques reduces the risk of or alleviates the severity of acute coronary syndrome.

8. The composition of Claim 7, wherein said acute coronary syndrome is acute myocardial infarction or unstable angina.

9. The composition of Claim 1, wherein said compound represented by formula (1) is 4,6-di-t-butyl-5-hydroxy-2,2-di-n-pentyl-2,3-dihydrobenzofuran.

10. A pharmaceutical composition for reducing the risk of or alleviating the severity of acute coronary syndrome, comprising a compound represented by formula (1): 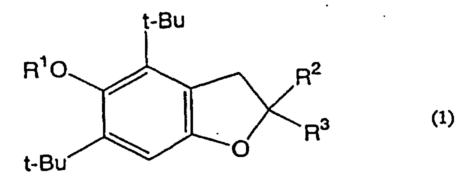 wherein
R¹ represents hydrogen atom, an acyl group or an arylalkoxycarbonyl group, and
R² and R³ are each independently a substituted or unsubstituted alkyl group, a substituted or unsubstituted alkenyl group, or a substituted or unsubstituted alkynyl group, or R² and R³ may combine to form a cycloalkyl group.

11. The composition of Claim 10, wherein said acute coronary syndrome is acute myocardial infarction or unstable angina.

12. The composition of Claim 10, wherein said compound represented by formula (1) is 4,6-di-t-butyl-5-hydroxy-2,2-di-n-pentyl-2,3-dihydrobenzofuran.

13. A pharmaceutical composition for decreasing an intracellular lipid content, comprising a compound represented by formula (1): 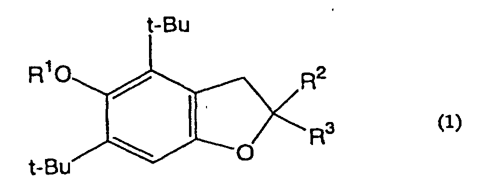 wherein
R¹ represents hydrogen atom, an acyl group or an arylalkoxycarbonyl group, and
R² and R³ are each independently a substituted or unsubstituted alkyl group, a substituted or unsubstituted alkenyl group, or a substituted or unsubstituted alkynyl group, or R² and R³ may combine to form a cycloalkyl group.

14. The composition of Claim 13, wherein said intracellular lipids are present in atherosclerotic plaques.

15. The composition of Claim 13, wherein said intracellular lipids are free cholesterol or cholesteryl esters.

16. The composition of Claim 13, wherein said decrease in intracellular lipid content results in prevention of the disruption of atherosclerotic plaques.

17. The composition of Claim 16, wherein said prevention of the disruption of atherosclerotic plaques reduces the risk of or alleviates the severity of acute coronary syndrome.

18. The composition of Claim 13, wherein said compound represented by formula (1) is 4,6-di-t-butyl-5-hydroxy-2,2-di-n-pentyl-2,3-dihydrobenzofuran.

19. A method for promoting the secretion of apolipoprotein E in a patient in need thereof which comprises administering to the patient an promoting effective amount of a compound represented by formula (1): 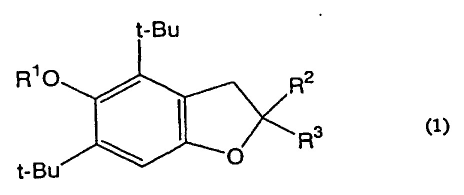 wherein
R¹ represents hydrogen atom, an acyl group or an arylalkoxycarbonyl group, and
R² and R³ are each independently a substituted or unsubstituted alkyl group, a substituted or unsubstituted alkenyl group, or a substituted or unsubstituted alkynyl group, or R² and R³ may combine to form a cycloalkyl group.

20. The method of Claim 19, wherein said secretion of apolipoprotein E increases a blood apolipoprotein E level or a topical apolipoprotein E level in an organ.

21. The method of Claim 20, wherein intracellular lipids are removed via the increase in a level of apolipoprotein E.

22. The method of Claim 21, wherein said intracellular lipids are present in atherosclerotic plaques.

23. The method of Claim 22, wherein said removal of intracellular lipids results in stabilization of atherosclerotic plaques for the prevention of the disruption of atherosclerotic plaques.

24. The method of Claim 21, wherein said intracellular lipids are free cholesterol or cholesteryl esters.

25. The method of Claim 23, wherein said prevention of the disruption of atherosclerotic plaques reduces the risk of or alleviates the severity of acute coronary syndrome.

26. The method of Claim 25, wherein said acute coronary syndrome is acute myocardial infarction or unstable angina.

27. The method of Claim 19, wherein said compound represented by formula (1) is 4,6-di-t-butyl-5-hydroxy-2,2-di-n-pentyl-2,3-dihydrobenzofuran.

28. A method for reducing the risk of or alleviating the severity of acute coronary syndrome in a patient in need thereof which comprises administering to the patient a therapeutically effective amount of a compound represented by formula (1): 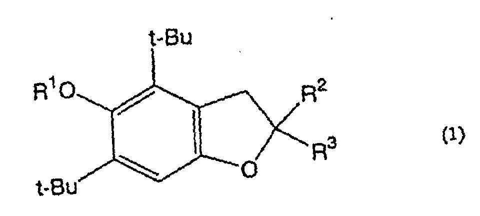 wherein
R¹ represents hydrogen atom, an acyl group or an arylalkoxycarbonyl group, and
R² and R³ are each independently a substituted or unsubstituted alkyl group, a substituted or unsubstituted alkenyl group, or a substituted or unsubstituted alkynyl group, or R² and R³ may combine to form a cycloalkyl group.

29. The method of Claim 28, wherein said acute coronary syndrome is acute myocardial infarction or unstable angina.

30. The method of Claim 28, wherein said compound represented by formula (1) is 4,6-di-t-butyl-5-hydroxy-2,2-di-n-pentyl-2,3-dihydrobenzofuran.

31. A method for decreasing an intracellular lipid content in a patient in need thereof which comprises administering to the patient a therapeutically effective amount of a compound represented by formula (1): 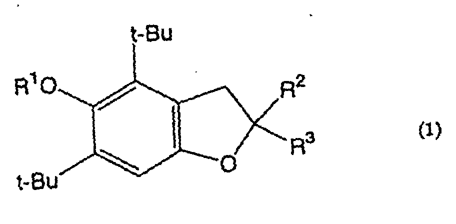 wherein
R¹ represents hydrogen atom, an acyl group or an arylalkoxycarbonyl group, and
R² and R³ are each independently a substituted or unsubstituted alkyl group, a substituted or unsubstituted alkenyl group, or a substituted or unsubstituted alkynyl group, or R² and R³ may combine to form a cycloalkyl group.

32. The method of Claim 31, wherein said intracellular lipids are present in atherosclerotic plaques.

33. The method of Claim 31, wherein said intracellular lipids are free cholesterol or cholesteryl esters.

34. The method of Claim 31, wherein said decrease in intracellular lipids results in prevention of the disruption of atherosclerotic plaques.

35. The method of Claim 34, wherein said prevention of the disruption of atherosclerotic plaques reduces the risk of or alleviates the severity of acute coronary syndrome.

36. The method of Claim 31, wherein said compound represented by formula (1) is 4,6-di-t-butyl-5-hydroxy-2,2-di-n-pentyl-2,3-dihydrobenzofuran.

37. Use of a compound represented by formula (1): 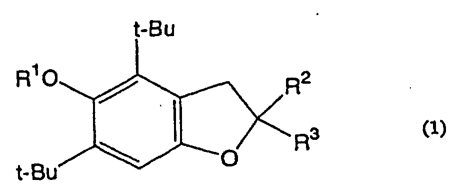 wherein
R¹ represents hydrogen atom, an acyl group or an arylalkoxycarbonyl group, and
R² and R³ are each independently a substituted or unsubstituted alkyl group, a substituted or unsubstituted alkenyl group, or a substituted or unsubstituted alkynyl group, or R² and R³ may combine to form a cycloalkyl group, in manufacturing a pharmaceutical composition for promoting the secretion of apolipoprotein E.

38. The use of Claim 37, wherein said secretion of apolipoprotein E increases a blood apolipoprotein E level or a topical apolipoprotein E level in an organ.

39. The use of Claim 38, wherein intracellular lipids are removed via the increase in a level of apolipoprotein E.

40. The use of Claim 39, wherein said intracellular lipids are present in atherosclerotic plaques.

41. The use of Claim 40, wherein said removal of intracellular lipids results in stabilization of atherosclerotic plaques for the prevention of the disruption of atherosclerotic plaques.

42. The use of Claim 39, wherein said intracellular lipids are free cholesterol or cholesteryl esters.

43. The use of Claim 41, wherein said prevention of the disruption of atherosclerotic plaques reduces the risk of or alleviates the severity of acute coronary syndrome.

44. The use of Claim 43, wherein said acute coronary syndrome is acute myocardial infarction or unstable angina.

45. The use of Claim 37, wherein said compound represented by formula (1) is 4,6-di-t-butyl-5-hydroxy-2,2-di-n-pentyl-2,3-dihydrobenzofuran.

46. Use of a compound represented by formula (1): 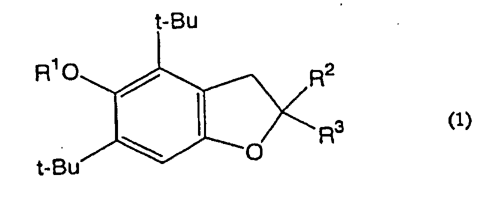 wherein
R¹ represents hydrogen atom, an acyl group or an arylalkoxycarbonyl group, and
R² and R³ are each independently a substituted or unsubstituted alkyl group, a substituted or unsubstituted alkenyl group, or a substituted or unsubstituted alkynyl group, or R² and R³ may combine to form a cycloalkyl group, in manufacturing a pharmaceutical composition for reducing the risk of or alleviating the severity of acute coronary syndrome.

47. The use of Claim 46, wherein said acute coronary syndrome is acute myocardial infarction or unstable angina.

48. The use of Claim 46, wherein said compound represented by formula (1) is 4,6-di-t-butyl-5-hydroxy-2,2-di-n-pentyl-2,3-dihydrobenzofuran.

49. Use of a compound represented by formula (1): 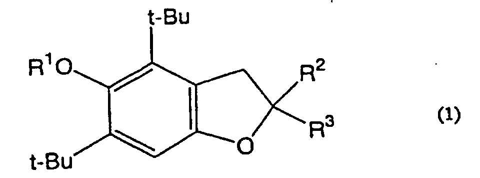 wherein
R¹ represents hydrogen atom, an acyl group or an arylalkoxycarbonyl group, and
R² and R³ are each independently a substituted or unsubstituted alkyl group, a substituted or unsubstituted alkenyl group, or a substituted or unsubstituted alkynyl group, or R² and R³ may combine to form a cycloalkyl group, in manufacturing a pharmaceutical composition for decreasing an intracellular lipid content.

50. The use of Claim 49, wherein said intracellular lipids are present in atherosclerotic plaques.

51. The use of Claim 49, wherein said intracellular lipids are free cholesterol or cholesteryl esters.

52. The use of Claim 49, wherein said decrease in intracellular lipid content results in prevention of the disruption of atherosclerotic plaques.

53. The use of Claim 52, wherein said prevention of the disruption of atherosclerotic plaques reduces the risk of or alleviates the severity of acute coronary syndrome.

54. The use of Claim 49, wherein said compound represented by formula (1) is 4,6-di-t-butyl-5-hydroxy-2,2-di-n-pentyl-2,3-dihydrobenzofuran.
